# EUROPEAN PATENT APPLICATION

(11) **EP 4 692 039 A1**
(43) Date of publication of application: **11.02.2026**
(21) Application number: 24850342.7
(22) Date of filing: 24.07.2024
(51) Int. Cl.: C07C 51/43, C07C 57/04, C07C 51/25

(54) **METHOD FOR PREPARATION OF ACRYLIC ACID**

(30) Priority: 22.09.2023 KR 20230126965
(71) Applicant: LG Chem, Ltd., Seoul 07336 (KR)
(72) Inventor: YOO, Sung Jin, Daejeon 34122 (KR); JANG, Kyung Soo, Daejeon 34122 (KR); LEE, Sung Kyu, Daejeon 34122 (KR)
(74) Representative: Goddar, Heinz J.
(86) International application number: PCT/KR2024/010678
(87) International publication number: WO 2025/063471

(57) **Abstract**

The present invention provides a method for preparing acrylic acid, the method including a multi-stage crystallization process performed by two or more crystallization units that are connected in series and each includes a crystallizer and a solid-liquid separator, wherein a first stage crystallization unit among the two or more crystallization units includes a crystallizer having a crystallization temperature of -20 to -10°C.

## Description

### [Technical Field]

### CROSS-REFERENCE TO RELATED APPLICATION

The present application claims the benefit of priority to Korean Patent Application No. 10-2023-0126965, filed on September 22, 2023, the entire contents of which are incorporated herein by reference.

### Technical Field

The present invention relates to a method for preparing acrylic acid, and more particularly, to a method for preparing high purity acrylic acid according to a continuous crystallization process.

### [Background Art]

Acrylic acid is generally prepared by a method of subjecting compounds such as propane, propylene, and acrolein to a gas phase oxidation reaction in the presence of a catalyst. For example, in the presence of a suitable catalyst in a reactor, propane, propylene, and the like are converted into acrylic acid via acrolein by the gas phase oxidation reaction, and at a rear end of the reactor, a mixed gas containing acrylic acid, unreacted propane or propylene, acrolein, an inert gas, carbon dioxide, water vapor, and various organic by-products (acetic acid, low boiling point by-products, high boiling point by-products, and the like) produced by the reaction is obtained.

The acrylic acid-containing mixed gas is brought into contact with an absorption solvent such as water in an absorption column and recovered as an acrylic acid solution. In addition, as a subsequent process for obtaining acrylic acid contained in the acrylic acid solution, processes such as extraction, distillation, and purification are generally involved.

However, since the specific heat of the absorption solvent such as water used in the absorption column is high, a significantly high energy usage has been required to separate by-products from the acrylic acid solution containing the absorption solvent through processes such as distillation. In addition, through processes such as high-temperature distillation, a polymer of acrylic acid, which is a reactive monomer, may be produced, and there is a problem of the need for periodic washing.

Meanwhile, although a crystallization process is involved to obtain acrylic acid contained in the acrylic acid solution, since a melting point of acetic acid, which is an organic by-product, is similar to that of the acrylic acid, when a large amount of acetic acid is introduced into the crystallization process, acrylic acid with a desired purity cannot be obtained by only a single-stage crystallization process, and it is difficult to obtain acrylic acid at a desired level.

Therefore, there is an urgent need to introduce a technique capable of increasing a final yield of acrylic acid while obtaining high purity acrylic acid from an acrylic acid solution.

### [Disclosure]

### [Technical Problem]

In order to solve the problems mentioned in the background art, an object of the present invention is to provide a method for preparing acrylic acid that may increase a final yield of acrylic acid and may ultimately obtain high purity acrylic acid in obtaining acrylic acid contained in the acrylic acid solution.

### [Technical Solution]

In one general aspect, a method for preparing acrylic acid includes a multi-stage crystallization process performed by two or more crystallization units that are connected in series and each includes a crystallizer and a solid-liquid separator, wherein in the crystallizer of each stage, acrylic acid contained in an acrylic acid solution introduced into the crystallizer of each stage is crystallized to obtain a suspension containing acrylic acid crystals, and the suspension is supplied to the solid-liquid separator of each stage; in the solid-liquid separator of each stage, the suspension is separated into a solid and a liquid to obtain a concentrated acrylic acid solution and a mother liquor; the mother liquor is divideddivided into a first mother liquor stream and a second mother liquor stream, the first mother liquor stream is circulated to a crystallizer at a front stage of a stage including the solid-liquid separator in which the mother liquor is separated or to a mother liquor treatment process, and the second mother liquor stream is circulated to a crystallizer at the stage including the solid-liquid separator in which the mother liquor is separated; the concentrated acrylic acid solution is introduced into a crystallizer at a rear stage of the stage including the solid-liquid separator in which the concentrated acrylic acid solution is separated; purified acrylic acid is obtained from the solid-liquid separator of the rearmost stage crystallization unit among the two or more crystallization units; and a first stage crystallization unit among the two or more crystallization units includes a crystallizer having a crystallization temperature of -20 to -10°C.

### [Advantageous Effects]

According to the method for preparing acrylic acid of the present invention, acrylic acid contained in the acrylic acid solution is crystallized by a multi-stage crystallization process performed by crystallization units that are connected in multiple stages, such that acrylic acid may be obtained with a high purity, and the final yield of acrylic acid may be improved.

In particular, the crystallization temperature in the crystallizer of the first stage crystallization unit among the two or more crystallization units is controlled to -20 to -10°C, such that the amount of acrylic acid crystallized in the crystallizer of the first stage crystallization unit may be the highest. Through this, the amount of mother liquor separated in the solid-liquid separator of the first stage crystallization unit may be reduced, and when a portion of the mother liquor separated from the first stage crystallization unit is circulated to a mother liquor treatment process, the amount of mother liquor that needs to be treated in the mother liquor treatment process is small, which may be preferable in terms of energy or economic feasibility. In addition, as the amount of crystallized acrylic acid increases, the final yield of purified acrylic acid may be satisfied.

### [Description of Drawings]

FIG. 1 is a process diagram showing a multi-stage crystallization process in a method for preparing acrylic acid according to an embodiment of the present invention.
FIGS. 2 to 4 are process diagrams showing a multi-stage crystallization process in a method for preparing acrylic acid according to comparative examples.

### [Best Mode]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

Hereinafter, the present invention will be described in more detail.

The term "stream" in the present invention may refer to a flow of a fluid in a process, and may also refer to a fluid flowing through a moving line (pipe) itself. Specifically, the "stream" may refer to both the fluid flowing through the pipe connecting respective devices to each other itself and the flow of the fluid. In addition, the fluid may refer to one or more of a gas, a liquid, and a solid.

A method for preparing acrylic acid according to an embodiment of the present invention may include a multi-stage crystallization process performed by two or more crystallization units that are connected in series and each includes a crystallizer and a solid-liquid separator, wherein in the crystallizer of each stage, acrylic acid contained in an acrylic acid solution introduced into the crystallizer of each stage is crystallized to obtain a suspension containing acrylic acid crystals, and the suspension is supplied to the solid-liquid separator of each stage; in the solid-liquid separator of each stage, the suspension is separated into a solid and a liquid to obtain a concentrated acrylic acid solution and a mother liquor; the mother liquor is divided into a first mother liquor stream and a second mother liquor stream, the first mother liquor stream is circulated to a crystallizer at a front stage of a stage including the solid-liquid separator in which the mother liquor is separated or to a mother liquor treatment process, and the second mother liquor stream is circulated to a crystallizer at the stage including the solid-liquid separator in which the mother liquor is separated; the concentrated acrylic acid solution is introduced into a crystallizer at a rear stage of the stage including the solid-liquid separator in which the concentrated acrylic acid solution is separated; purified acrylic acid is obtained from the solid-liquid separator of the rearmost stage crystallization unit among the two or more crystallization units; and the two or more crystallization units include a crystallizer having a crystallization temperature of -20 to -10°C.

The method for preparing acrylic acid according to an embodiment of the present invention may include a multi-stage crystallization process performed by two or more crystallization units that are connected in series and each includes a crystallizer and a solid-liquid separator.

Specifically, in the multi-stage crystallization process, the two or more crystallization units may include multi-stage crystallizers connected in multiple stages, and the crystallization processes performed in the respective crystallization units of the stages may be performed continuously. Each of the crystallization units of the respective stages may include a crystallizer and a solid-liquid separator.

The crystallizer may be a device that crystallizes a specific substance into a solid by using a difference in solubility of the substance according to a temperature in a liquid mixture, and the solid-liquid separator may be a device that separates a solid-liquid mixture supplied to the solid-liquid separator into a solid phase and a liquid phase.

Meanwhile, when crystallization of the acrylic acid solution is performed by a single-stage crystallization process, acrylic acid crystals cannot be obtained with a desired purity by only the single-stage crystallization process because a content of impurities in the acrylic acid solution is high.

However, as in the present invention, when the crystallization process is performed in multiple stages, the acrylic acid solution is concentrated and crystallized in multiple stages, such that the final yield of acrylic acid may be satisfied, and the purity of the acrylic acid may be improved.

In the method for preparing acrylic acid according to an embodiment of the present invention, in the crystallizer of each stage, acrylic acid contained in an acrylic acid solution introduced into the crystallizer of each stage may be crystallized to obtain a suspension containing acrylic acid crystals, and the suspension may be supplied to the solid-liquid separator of each stage.

Specifically, the acrylic acid solution introduced into the crystallizer of the first stage crystallization unit among the crystallizers of the respective stages may be a crude acrylic acid aqueous solution containing crude acrylic acid. In this case, the crude acrylic acid may be acrylic acid before crystallization. Meanwhile, the first stage crystallization unit may be a crystallization unit located at the frontmost stage among the two or more crystallization units, and may be located at the stage including the crystallizer into which the crude acrylic acid aqueous solution is introduced.

The crude acrylic acid aqueous solution may be prepared as follows.

First, a reactant containing a gas containing oxygen and a raw material compound may be supplied to a reactor equipped with a catalyst, and a gas phase oxidation reaction may be performed in the presence of the catalyst in the reactor, thereby obtaining a mixed gas containing acrylic acid. In this case, the acrylic acid may be crude acrylic acid, and the gas containing oxygen may be air.

The raw material compound may be one or more compounds selected from the group consisting of propane, propylene, butane, i-butylene, t-butylene, and acrolein, and specifically, the raw material compound may include propylene.

Therefore, the mixed gas containing acrylic acid of the present invention may be a reaction product resulting from a gas phase oxidation reaction of a reactant containing air and a raw material compound in the reactor. Specifically, the mixed gas may contain acrylic acid, an unreacted raw material compound, acrolein, an inert gas, carbon monoxide, carbon dioxide, and various organic by-products (acetic acid, low boiling point by-products, high boiling point by-products, and the like). Here, the "low boiling point by-products (light ends)" or the "high boiling point by-products (heavies)" refer to a type of by-product that may be produced in a process of preparing desired acrylic acid, and may be a compound having a molecular weight smaller or larger than that of acrylic acid.

The acrylic acid solution introduced into the crystallizer of the first stage crystallization unit may be obtained by bringing a mixed gas containing acrylic acid into contact with an absorption solvent.

Specifically, the acrylic acid solution may be obtained through an absorption process in which the mixed gas containing acrylic acid is brought into contact with water, which is an absorption solvent. In this case, considering the efficiency of the absorption process, the mixed gas may be supplied to a lower portion of an absorption column where the absorption process is performed, and the absorption solvent, specifically, water, may be supplied to an upper portion of the absorption column.

As such, the acrylic acid solution obtained by the absorption process, that is, the crude acrylic acid aqueous solution, may be introduced into the crystallizer of the first stage crystallization unit, and the crystallization process may be performed.

According to the present invention, a content of the acrylic acid contained in the acrylic acid solution introduced into the crystallizer of the first stage crystallization unit may be 80 to 95 wt%, and specifically, may be 82 wt% to 90 wt%. Depending on the content of the acrylic acid in the acrylic acid solution introduced into the crystallizer of the first stage crystallization unit, the temperature at which acrylic acid is crystallized in the crystallizer of each stage and the amount of acrylic acid crystals produced according to the temperature may vary.

More specifically, when the content of the acrylic acid in the acrylic acid solution introduced into the crystallizer of the first stage crystallization unit is less than 80 wt%, since the content of the acrylic acid in the acrylic acid solution is low, when the acrylic acid solution is supplied to the crystallizer, the amount of acrylic acid crystals obtained by crystallization may also be small, and the process performed to satisfy the purity and yield of acrylic acid at the desired level in the present invention may become complicated. Meanwhile, when the content of the acrylic acid in the acrylic acid solution introduced into the crystallizer of the first stage crystallization unit exceeds 95 wt%, an additional purification process may be required to obtain such an acrylic acid solution, which may be undesirable from the viewpoint of economic feasibility such as the process facility costs and device costs for performing the purification process. In addition, when the purification process is performed, energy usage may increase excessively.

Meanwhile, as described above, the crude acrylic acid aqueous solution obtained as described above may be introduced into the crystallizer of the first stage crystallization unit as an acrylic acid solution. Meanwhile, the concentrated acrylic acid solution separated in the solid-liquid separator described below, specifically, the solid-liquid separator of the front stage, may be introduced as the acrylic acid solution into each of the crystallizers of the crystallization units subsequent to the first stage crystallization unit, that is, each of the crystallizers of the crystallization units from the second stage.

Here, the crystallization unit subsequent to the first stage crystallization unit may refer to any crystallization unit subsequent to the first stage crystallization unit among the two or more crystallization units. For example, when the crystallization units are included in four stages in total, the crystallization units subsequent to a first stage crystallization unit may be a second stage crystallization unit, a third stage crystallization unit, and a fourth stage crystallization unit.

Accordingly, as the crystallization unit is located at a further rear stage from the crystallization unit subsequent to the first stage crystallization unit, the content of the acrylic acid contained in the concentrated acrylic acid solution introduced into the crystallizer of each crystallization unit may increase.

Meanwhile, through the crystallization process performed in the crystallizer of each stage, the acrylic acid contained in the acrylic acid solution is crystallized, thereby obtaining a suspension containing acrylic acid crystals. Thereafter, the suspension obtained in the crystallizer of each stage may be supplied to the solid-liquid separator of the stage including the crystallizer in which the suspension is obtained.

According to an embodiment of the present invention, a ratio of the mass of the acrylic acid crystals contained in the suspension to the mass of the suspension discharged from the crystallizer of each stage may be 10 to 40 wt%, and specifically, 20 to 35 wt%. A ratio of the mass of the acrylic acid crystals contained in the suspension to the total mass of the suspension discharged from the crystallizer of each stage may have the same meaning as a ratio of a mass flow rate of the crystallized acrylic acid contained in the suspension to a mass flow rate of the suspension supplied to the solid-liquid separator of each stage.

More specifically, when the ratio of the mass of the acrylic acid crystals contained in the suspension to the mass of the suspension discharged from the crystallizer of each stage, that is, an acrylic acid crystal concentration (acrylic acid crystal content) in the suspension, is less than 10 wt%, since a relatively small amount of acrylic acid is crystallized in the crystallizer of each stage, the total number of times the multi-stage crystallization process is performed and the throughput may be increased in order to satisfy the desired level of acrylic acid production. When the acrylic acid crystal concentration in the suspension exceeds 40 wt%, the content of the acrylic acid crystals, which are solids, in the suspension increases, which may make it difficult to mix the suspension inside the crystallizer of each stage. Accordingly, it may also be difficult to move the suspension to the solid-liquid separator, which may make it difficult to perform the crystallization process.

According to the method for preparing acrylic acid according to an embodiment of the present invention, in the solid-liquid separator of each stage, the suspension may be separated into a solid and a liquid to obtain a concentrated acrylic acid solution and a mother liquor. Specifically, in the solid-liquid separator of each stage, the suspension obtained in the crystallizer of each stage may be separated into a solid and a liquid to obtain solid acrylic acid crystals and a liquid mother liquor. The mother liquor may be the remainder obtained by separating acrylic acid crystals from the suspension, and may contain uncrystallized acrylic acid, water, and acetic acid.

Furthermore, in the solid-liquid separator of each stage, the concentrated acrylic acid may be obtained by separating the suspension into a solid and a liquid to obtain acrylic acid crystals and melting the acrylic acid crystals. That is, the concentrated acrylic acid solution may be obtained by melting the acrylic acid crystals by heat. For example, the molten acrylic acid crystals may be introduced into a crystallizer at a rear stage as the concentrated acrylic acid solution.

More specifically, the melting may be a physical process of transitioning from a solid phase to a liquid phase, and even when the melting of the acrylic acid crystals is performed, the number of moles of acrylic acid contained in the acrylic acid crystals may not change. In this case, a separate heat supply device, for example, a heat exchanger, may be additionally provided to melt the acrylic acid crystals. The concentrated acrylic acid solution may contain acrylic acid, and in addition, may contain impurities (water, acetic acid, and the like) contained in the acrylic acid crystals and a trace amount of mother liquor that is not separated in the solid-liquid separator of each stage and remains in the acrylic acid crystals.

It is preferable to melt the acrylic acid crystals separated in the solid-liquid separator of each of the two or more crystallization units in order to introduce the acrylic acid crystals into the crystallizer of the crystallization unit at a rear stage of the stage including the solid-liquid separator in which the acrylic acid crystals are separated, or to obtain the acrylic acid crystals as a product. That is, the melting of the acrylic acid crystals may be performed for movement of the acrylic acid crystals in the multi-stage crystallization process because the acrylic acid crystals are in a solid phase.

According to the method for preparing acrylic acid according to an embodiment of the present invention, the mother liquor may be branched into a first mother liquor stream and a second mother liquor stream, the first mother liquor stream may be circulated to a crystallizer at a front stage of a stage including the solid-liquid separator in which the mother liquor is separated or to a mother liquor treatment process, and the second mother liquor stream may be circulated to a crystallizer at the stage including the solid-liquid separator in which the mother liquor is separated.

Specifically, the mother liquor is a mother liquor separated from the solid-liquid separator of each stage, and may be divided into a first mother liquor stream and a second mother liquor stream.

Thereafter, the first mother liquor stream may be circulated to the mother liquor treatment process or a crystallizer at a front stage of a stage including the solid-liquid separator from which the first mother liquor stream is derived. Specifically, the first mother liquor stream divided from the mother liquor obtained in the solid-liquid separator of the first stage crystallization unit may be circulated to the mother liquor treatment process. Meanwhile, the first mother liquor stream divided from the mother liquor obtained in the solid-liquid separator of the crystallization unit subsequent to the first stage crystallization unit may be circulated to a crystallizer at a front stage of a stage including the solid-liquid separator in which the mother liquor is obtained.

According to the present invention, the mother liquor treatment process may include one or more of a purge and an acrylic acid purification process. Specifically, the mother liquor treatment process may be a process for treating the first mother liquor stream in the mother liquor separated in the solid-liquid separator of the first stage crystallization unit.

More specifically, the purge may be performed when a content of acrylic acid in the first mother liquor stream branched from the solid-liquid separator of the first stage crystallization unit is relatively low, and may be a process of discharging and discarding the first mother liquor stream divided from the solid-liquid separator of the first stage crystallization unit to the outside of the system.

Meanwhile, the acrylic acid purification process may be performed when the content of the acrylic acid in the first mother liquor stream divided from the solid-liquid separator of the first stage crystallization unit is relatively high, and may be a process of distilling the first mother liquor stream divided from the solid-liquid separator of the first stage crystallization unit and separating acrylic acid.

Meanwhile, the second mother liquor stream may be circulated to the crystallizer at the stage including the solid-liquid separator from which the second mother liquor stream is divided.

According to an embodiment of the present invention, a mass flow rate ratio of the first mother liquor stream and the second mother liquor stream may be 0.5:9.5 to 9.5:0.5, and specifically, 1:9 to 9:1. The mass flow rate ratio of the first mother liquor stream and the second mother liquor stream may refer to a mass flow rate ratio of the first mother liquor stream and the second mother liquor stream divided from the solid-liquid separator of each stage. When the mass flow rate ratio of the first mother liquor stream and the second mother liquor stream is controlled to be within the above range, the acrylic acid crystal concentration in the suspension described above may be controlled to 10 to 40 wt%. Through this, the fluidity of the suspension may be maximized, and the flowability of the stream may be flexible when performing the multi-stage crystallization process.

According to the method for preparing acrylic acid according to an embodiment of the present invention, the concentrated acrylic acid solution may be introduced into a crystallizer at a rear stage of the stage including the solid-liquid separator in which the concentrated acrylic acid solution is separated.

Specifically, the concentrated acrylic acid solution is obtained by melting the acrylic acid crystals separated in the solid-liquid separator of each stage. The concentrated acrylic acid solution may be introduced into a crystallizer at a rear stage of the stage including the solid-liquid separator in which the concentrated acrylic acid solution is separated. Therefore, as described above, the concentrated acrylic acid solution may be introduced as an acrylic acid solution into a crystallizer of a crystallization unit subsequent to the first stage crystallization unit.

For example, when the crystallization units are included in three stages in total, a concentrated acrylic acid solution separated in a solid-liquid separator of a second stage crystallization unit may be introduced into a crystallizer of a third stage crystallization unit.

According to the method of preparing acrylic acid according to an embodiment of the present invention, purified acrylic acid may be obtained from the solid-liquid separator of the rearmost stage crystallization unit among the two or more crystallization units.

As described above, the acrylic acid crystals separated in the solid-liquid separator of each stage may be melted to obtain a concentrated acrylic acid solution, and the concentrated acrylic acid solution may be introduced into a crystallizer at a rear stage of the stage including the solid-liquid separator from which the concentrated acrylic acid solution is derived.

However, since a crystallization unit at a rear stage of the rearmost stage crystallization unit does not exist and acrylic acid needs to be finally obtained, the acrylic acid crystals separated in the solid-liquid separator of the rearmost stage crystallization unit may be melted to obtain purified acrylic acid, which is a product of the present invention. The rearmost stage crystallization unit may refer to a crystallization unit located at the rearmost stage among the two or more crystallization units connected in multiple stages.

According to the method for preparing acrylic acid of the present invention, the first stage crystallization unit among the two or more crystallization units may include a crystallization unit having a crystallization temperature of -20 to -10°C. In this case, the crystallization temperature may refer to an operating temperature of the crystallizer for crystallizing the acrylic acid solution introduced into the crystallizer of each stage into acrylic acid crystals. When the crystallization temperature in the crystallizer included in the first stage crystallization unit is within the above range, the yield of acrylic acid in the crystallizer of the first stage crystallization unit may be the highest. In this case, the yield of acrylic acid may refer to the mass of acrylic acid crystallized in the crystallizer of each stage relative to the mass of acrylic acid contained in the acrylic acid solution introduced into the crystallizer of each stage.

Through this, the amount of the mother liquor separated in the solid-liquid separator of the first stage crystallization unit may be reduced, and when a portion of the small amount of mother liquor is circulated to the mother liquor treatment process, it is preferable from the viewpoint of economic feasibility or energy usage. For example, when a large amount of mother liquor is purged, the cost of loss of acrylic acid contained in the mother liquor is excessively incurred, and when a large amount of mother liquor is circulated to the acrylic acid purification process, the energy used to purify a large amount of mother liquor in the acrylic acid purification process may increase significantly. Therefore, it is preferable to reduce the amount of the mother liquor circulated to the mother liquor treatment process.

In addition, the acrylic acid crystals separated in the solid-liquid separator of the first stage crystallization unit are melted, the molten acrylic acid crystals are introduced into the crystallizer of the crystallization unit at the rear stage of the first stage crystallization unit, and a multi-stage crystallization process is performed, such that the final yield of acrylic acid may be satisfied, and purified acrylic acid may be obtained with a high purity. In this case, the final yield of acrylic acid may refer to a ratio of the mass of the acrylic acid contained in the concentrated acrylic acid solution separated in the solid-liquid separator of the rearmost stage crystallization unit to the mass of the acrylic acid in the acrylic acid solution introduced into the crystallizer of the first stage crystallization unit.

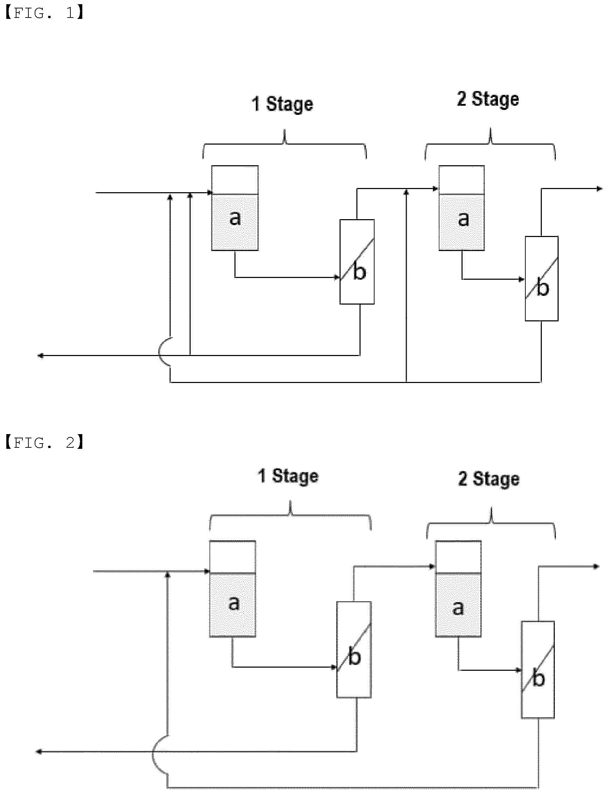

Hereinafter, a process that may be included in an embodiment of the present invention will be described with reference to FIG. 1.

According to an embodiment of the present invention, the two or more crystallization units are including a first stage crystallization unit and a second stage crystallization unit included in two stages in total, a crystallization temperature in a crystallizer (a) of a first stage crystallization unit may be -20 to -10°C, and a crystallization temperature in a crystallizer (a) of a second stage crystallization unit may be 0 to 12°C. Specifically, the crystallization temperature in the crystallizer (a) of the first stage crystallization unit may be -14 to -9°C, and the crystallization temperature in the crystallizer (a) of the second stage crystallization unit may be 4 to 9°C.

More specifically, as the crystallization temperature in the crystallizer (a) of the first stage crystallization unit is lower, the yield of acrylic acid in the crystallizer (a) of the first stage crystallization unit may be maximized, but since crystals of acrylic acid and water are simultaneously produced at a temperature below a eutectic point of acrylic acid and water, it is preferable that the crystallization temperature in the crystallizer (a) of the first stage crystallization unit is a temperature higher than the eutectic point. Therefore, the crystallization temperature in the crystallizer (a) of the first stage crystallization unit may be a temperature that is close to the eutectic point of acrylic acid and water contained in the acrylic acid solution introduced into the crystallizer of the first stage crystallization unit described above, but is higher than the eutectic point, when the content of acrylic acid contained in the acrylic acid solution is 80 to 95 wt%.

Furthermore, when the crystallization temperature in the crystallizer (a) of the first stage crystallization unit is within the above range, the yield of acrylic acid in the crystallizer (a) of the first stage crystallization unit may be the highest. This is because, as the crystallization temperature is close to the eutectic point, the mass of the solid produced in the crystallizer may increase compared to the liquid. As such, in the crystallizer (a) of the first stage crystallization unit, a large number of acrylic acid crystals are produced, and the acrylic acid crystals are melted and subjected to a multi-stage crystallization process through the crystallization unit subsequent to the first stage crystallization unit, such that the final yield of purified acrylic acid may be increased.

Meanwhile, when the crystallization temperature in the crystallizer (a) of the second stage crystallization unit is within the above range, first and second mother liquor streams divided from the solid-liquid separator (b) of the second stage crystallization unit are supplied to the crystallizers (a) of the first stage and second stage crystallization units, respectively, to control an acrylic acid crystal concentration in the suspension discharged from the crystallizers (a) of the first stage and second stage crystallization units to 10 to 40 wt%. In addition, it is preferable to control the crystallization temperature in the crystallizer of the second stage crystallization unit to be within the above range in terms of the refrigerant (utility) usage for the crystallization process and the purity of acrylic acid.

According to an embodiment of the present invention, a mass flow rate ratio of the first mother liquor stream and the second mother liquor stream divided from the solid-liquid separator (b) of the first stage crystallization unit may be 0.5:9.5 to 1:1, and a mass flow rate ratio of the first mother liquor stream and the second mother liquor stream divided from the solid-liquid separator (b) of the second stage crystallization unit may be 1:1 to 9:1. In this case, the acrylic acid crystal concentration in the suspension discharged from the crystallizer (a) of each stage may be controlled to 10 to 40 wt%. Through this, when the multi-stage crystallization process is performed, the flows of the streams in the crystallization units or between the crystallization units may be performed continuously. In this case, the stream may contain, for example, the suspension.

The ranges of the mass flow rate ratio of the first mother liquor stream and the second mother liquor stream branched from the solid-liquid separator (b) of the first stage crystallization unit and the mass flow rate ratio of the first mother liquor stream and the second mother liquor stream divided from the solid-liquid separator (b) of the second stage crystallization unit may be determined according to the crystallization temperature in the crystallizer (a) of each stage and the content of acrylic acid crystals contained in the suspension discharged from the crystallizer (a) of each stage. Therefore, the mass flow rate ratio in each of the first stage and second stage crystallization units may be a mass flow rate ratio controlled when the crystallization temperature in the crystallizer (a) of the first stage crystallization unit is -20 to -10°C and the crystallization temperature in the crystallizer (a) of the second stage crystallization unit is 0 to 12°C.

According to an embodiment, the first stage crystallization unit may include an auxiliary crystallizer disposed at a front stage of the crystallizer. Specifically, the auxiliary crystallizer is a separate device from the crystallizer, and the auxiliary crystallizer and the crystallizer may be connected to each other. For example, the acrylic acid solution, that is, the crude acrylic acid aqueous solution, may be supplied to the auxiliary crystallizer, and a suspension obtained in the auxiliary crystallizer may be supplied to the crystallizer connected subsequently to the auxiliary crystallizer.

When the first stage crystallization unit further includes the auxiliary crystallizer, a temperature gradient may be formed through the auxiliary crystallizer and the crystallizer, and the crystallization process may be performed in the first stage crystallization unit.

Specifically, a crystallization temperature in the auxiliary crystallizer may be -5 to 5°C. More specifically, when the crystallization temperature in the auxiliary crystallizer is within the above range, the temperature of the crude acrylic acid aqueous solution supplied to the auxiliary crystallizer and the crystallization temperature in the auxiliary crystallizer may not differ drastically. This is preferable in terms of the purity of the acrylic acid crystals produced.

Hereinafter, the present invention will be described in more detail with reference to examples. However, the following examples are provided for illustrating the present invention. It is apparent to those skilled in the art that various modifications and alterations may be made without departing from the scope and spirit of the present invention, and the scope of the present invention is not limited thereto.

### Examples

### Example 1

According to the process diagram illustrated in FIG. 1, an acrylic acid preparation process was simulated using Aspen Plus simulator available from Aspen Technology, Inc. As a crystallizer model, a model reflecting distribution coefficient values of water and acetic acid from Solid-Liquid Equilibrium (SLE) data of acrylic acid and water, acrylic acid and acetic acid, and water and acetic acid, and crystallization experiment results of acrylic acid, water, and acetic acid was used.

Specifically, an acrylic acid solution containing acrylic acid (85 wt%), acetic acid (3 wt%), and water (12 wt%) was prepared, and the acrylic acid solution was introduced into a multi-stage crystallization process performed by crystallization units connected in two stages in total.

Specifically, the acrylic acid solution was supplied to a crystallizer (a) of a first stage crystallization unit and a crystallization process was performed. Through this, a suspension containing acrylic acid crystals was obtained. At this time, the crystallization temperature in the crystallizer (a) of the first stage crystallization unit was -13°C. The ratio of the mass of the acrylic acid crystals contained in the suspension to the mass of the suspension discharged from the crystallizer (a) of the first stage crystallization unit was 30 wt%.

Thereafter, the obtained suspension was supplied from the crystallizer (a) of the first stage crystallization unit to a solid-liquid separator (b) of the first stage crystallization unit. In the solid-liquid separator (b) of the first stage crystallization unit, the suspension obtained in the crystallizer (a) of the first stage crystallization unit was separated into acrylic acid crystals and a mother liquor. At this time, in the solid-liquid separator (b) of the first stage crystallization unit, solid-liquid separation of the suspension was performed through filtration.

The mother liquor separated in the solid-liquid separator (b) of the first stage crystallization unit was divided into a first mother liquor stream and a second mother liquor stream, and the first mother liquor stream and the second mother liquor stream were circulated to a mother liquor treatment process and the crystallizer (a) of the first stage crystallization unit, respectively. At this time, the mass flow rate ratio of the first mother liquor stream and the second mother liquor stream branched from the solid-liquid separator (b) of the first stage crystallization unit was 1.2:8.8.

Meanwhile, the acrylic acid crystals separated in the solid-liquid separator (b) of the first stage crystallization unit were melted to obtain a concentrated acrylic acid solution. The concentrated acrylic acid solution derived from the solid-liquid separator (b) of the first stage crystallization unit was supplied to a crystallizer (a) of a second stage crystallization unit.

Through the crystallization process performed in the crystallizer (a) of the second stage crystallization unit, a suspension containing acrylic acid crystals was obtained. At this time, the crystallization temperature in the crystallizer (a) of the second stage crystallization unit was 8°C. The ratio of the mass of the acrylic acid crystals contained in the suspension to the mass of the suspension discharged from the crystallizer (a) of the second stage crystallization unit was 30 wt%.

Thereafter, the obtained suspension was supplied from the crystallizer (a) of the second stage crystallization unit to a solid-liquid separator (b) of the second stage crystallization unit. In the solid-liquid separator (b) of the second stage crystallization unit, the suspension obtained in the crystallizer (a) of the second stage crystallization unit was separated into acrylic acid crystals and a mother liquor. At this time, in the solid-liquid separator (b) of the second stage crystallization unit, solid-liquid separation of the suspension was performed through filtration.

The mother liquor separated in the solid-liquid separator (b) of the second stage crystallization unit was divided into a first mother liquor stream and a second mother liquor stream, and the first mother liquor stream and the second mother liquor stream were circulated to the crystallizers (a) of the first stage crystallization unit and the second stage crystallization unit, respectively. At this time, the mass flow rate ratio of the first mother liquor stream and the second mother liquor stream branched from the solid-liquid separator (b) of the second stage crystallization unit was 6.7:3.3.

Meanwhile, the acrylic acid crystals separated in the solid-liquid separator (b) of the second stage crystallization unit were melted to obtain a concentrated acrylic acid solution as purified acrylic acid.

As a result, acrylic acid (99.0 wt%), acetic acid (0.61 wt%), and water (0.37 wt%) were contained in the purified acrylic acid. In addition, the ratio of the mass of acrylic acid contained in the acrylic acid crystals separated in the solid-liquid separator (b) of the second stage crystallization unit to the mass of acrylic acid contained in the acrylic acid solution supplied to the crystallizer (a) of the first stage crystallization unit, that is, the final yield of acrylic acid, was 69.8%.

### Example 2

Compared to Example 1, in Example 2, the crystallization temperature in the crystallizer (a) of the second stage crystallization unit was -2°C, the mass flow rate ratio of the first mother liquor stream and the second mother liquor stream divided from the solid-liquid separator of the first stage crystallization unit was 2.4:7.6, and the mass flow rate ratio of the first mother liquor stream and the second mother liquor stream branched from the solid-liquid separator (b) of the second stage crystallization unit was 0.5:9.5.

As a result, acrylic acid (97.5 wt%), acetic acid (1.2 wt%), and water (1.3 wt%) were contained in the purified acrylic acid, and the final yield of acrylic acid was 72.5%.

### Comparative Examples

### Comparative Example 1

According to the process diagram illustrated in FIG. 2, an acrylic acid preparation process was simulated using Aspen Plus simulator available from Aspen Technology, Inc.

In Comparative Example 1, the mother liquor separated in each of solid-liquid separators (b) of a first stage crystallization unit and a second stage crystallization unit was not divided, and the mother liquors were supplied to a mother liquor treatment process and a crystallizer (a) of the first stage crystallization unit, respectively.

Specifically, the acrylic acid solution prepared with the same composition as that of Example 1 was introduced into the crystallizer (a) of the first stage crystallization unit to obtain a suspension containing acrylic acid crystals. The acrylic acid solution had the same composition as that of Example 1. The crystallization temperature in the crystallizer (a) of the first stage crystallization unit was -0.2°C. The obtained suspension was supplied from the crystallizer (a) of the first stage crystallization unit to a solid-liquid separator (b) of the first stage crystallization unit. At this time, the ratio of the mass of the acrylic acid crystals contained in the suspension to the mass of the suspension discharged from the crystallizer (a) of the second stage crystallization unit was 30 wt%.

In the solid-liquid separator (b) of the first stage crystallization unit, the suspension obtained in the crystallizer (a) of the first stage crystallization unit was separated into acrylic acid crystals and a mother liquor. At this time, in the solid-liquid separator (b) of the first stage crystallization unit, solid-liquid separation of the suspension was performed through filtration. The acrylic acid crystals separated in the solid-liquid separator (b) of the first stage crystallization unit were melted to obtain a concentrated acrylic acid solution. The concentrated acrylic acid solution derived from the solid-liquid separator (b) of the first stage crystallization unit was supplied to the crystallizer (a) of the second stage crystallization unit, and the mother liquor separated in the solid-liquid separator (b) of the first stage crystallization unit was introduced into a mother liquor treatment process.

In the crystallizer (a) of the second stage crystallization unit, the concentrated acrylic acid solution derived from the solid-liquid separator (b) of the first stage crystallization unit was crystallized to obtain a suspension containing acrylic acid crystals. At this time, the crystallization temperature in the crystallizer (a) of the second stage crystallization unit was 11.1°C.

The obtained suspension was supplied from the crystallizer (a) of the second stage crystallization unit to the solid-liquid separator (b) of the second stage crystallization unit. In the solid-liquid separator (b) of the second stage crystallization unit, the suspension obtained in the crystallizer (a) of the second stage crystallization unit was separated into acrylic acid crystals and a mother liquor. At this time, in the solid-liquid separator (b) of the second stage crystallization unit, solid-liquid separation of the suspension was performed through filtration. The separated mother liquor was supplied from the solid-liquid separator (b) of the second stage crystallization unit to the crystallizer (a) of the first stage crystallization unit. The acrylic acid crystals separated in the solid-liquid separator (b) of the second stage crystallization unit were melted to obtain a concentrated acrylic acid solution as purified acrylic acid.

As a result, acrylic acid (99.6 wt%), acetic acid (0.29 wt%), and water (0.15 wt%) were contained in the purified acrylic acid, and the final yield of acrylic acid was 13.8%.

Compared to Example 1, Comparative Example 1 was a case in which the mother liquors separated in the solid-liquid separators of the first stage and second stage crystallization units were not divided, and it was confirmed that the final yield of acrylic acid was the lowest.

### Comparative Example 2

Compared to Comparative Example 1, in Comparative Example 2, the crystallization temperature in the crystallizer (a) of the first stage crystallization unit was -4.5°C, and the ratio of the mass of acrylic acid crystals contained in the suspension to the mass of the suspension discharged from the crystallizer (a) of the first stage crystallization unit was 50 wt%.

As a result, mixing was not performed in the crystallizer (a) of the first stage crystallization unit, and thus, the crystallization process was not performed and acrylic acid was not obtained.

### Comparative Example 3

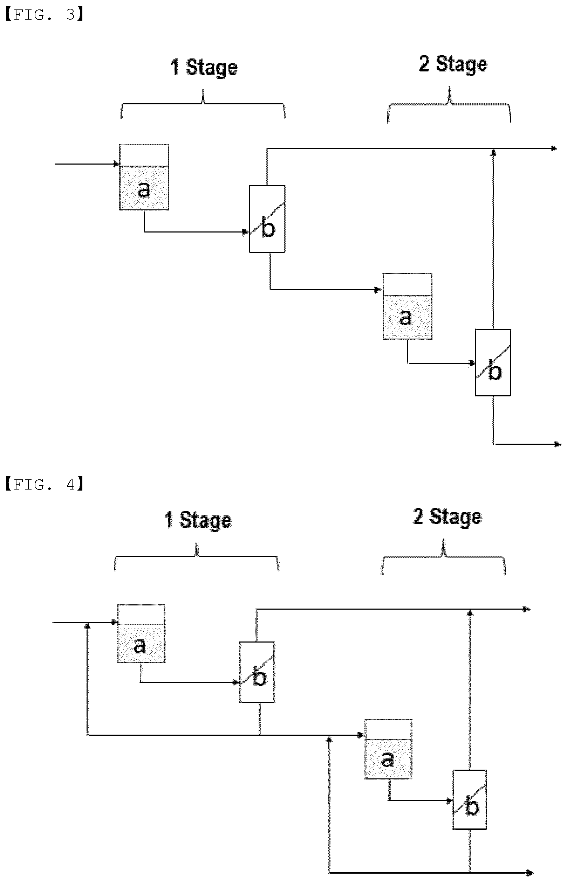

According to the process diagram illustrated in FIG. 3, an acrylic acid preparation process was simulated using Aspen Plus simulator available from Aspen Technology, Inc.

Specifically, an acrylic acid solution with the same composition as that of Example 1 was introduced into a crystallizer (a) of a first stage crystallization unit. In the crystallizer (a) of the first stage crystallization unit, the acrylic acid solution was crystallized to obtain a suspension containing acrylic acid crystals. At this time, the crystallization temperature in the crystallizer (a) of the first stage crystallization unit was -2.6°C. The ratio of the mass of the acrylic acid crystals contained in the suspension to the mass of the suspension discharged from the crystallizer (a) of the first stage crystallization unit was 30 wt%.

The obtained suspension was supplied from the crystallizer (a) of the first stage crystallization unit to a solid-liquid separator (b) of the first stage crystallization unit. In the solid-liquid separator (b) of the first stage crystallization unit, the suspension obtained in the crystallizer (a) of the first stage crystallization unit was separated into acrylic acid crystals and a mother liquor. At this time, in the solid-liquid separator (b) of the first stage crystallization unit, solid-liquid separation of the suspension was performed through filtration. Meanwhile, the acrylic acid crystals separated in the solid-liquid separator (b) of the first stage crystallization unit were melted to obtain a concentrated acrylic acid solution.

The separated mother liquor was supplied from the solid-liquid separator (b) of the first stage crystallization unit to a crystallizer (a) of a second stage crystallization unit and crystallized to obtain a suspension containing acrylic acid crystals. At this time, the crystallization temperature in the crystallizer (a) of the second stage crystallization unit was -7.7°C. The ratio of the mass of the acrylic acid crystals contained in the suspension to the mass of the suspension discharged from the crystallizer (a) of the second stage crystallization unit was 30 wt%.

The obtained suspension was supplied from the crystallizer (a) of the second stage crystallization unit to a solid-liquid separator (b) of the second stage crystallization unit, and acrylic acid crystals and a mother liquor were separated. At this time, in the solid-liquid separator (b) of the second stage crystallization unit, solid-liquid separation of the suspension was performed through filtration. The mother liquor separated in the solid-liquid separator (b) of the second stage crystallization unit was discharged.

Meanwhile, the acrylic acid crystals separated in the solid-liquid separator (b) of the second stage crystallization unit were melted to obtain a concentrated acrylic acid solution, a mixed stream was formed by the concentrated acrylic acid solution separated in the solid-liquid separator (b) of the second stage crystallization unit and the concentrated acrylic acid solution separated in the solid-liquid separator (b) of the first stage crystallization unit, and the mixed stream was obtained as purified acrylic acid.

Acrylic acid (97.1 wt%), acetic acid (1.1 wt%), and water (1.7 wt%) were contained in the purified acrylic acid, and the final yield of acrylic acid was 59.6%.

As a result, in Comparative Example 3, it was confirmed that the purity of purified acrylic acid and the final yield of acrylic acid were reduced compared to the examples.

### Comparative Example 4

According to the process diagram illustrated in FIG. 4, an acrylic acid preparation process was simulated using Aspen Plus simulator available from Aspen Technology, Inc.

Specifically, an acrylic acid solution with the same composition as that of Example 1 was introduced into a crystallizer (a) of a first stage crystallization unit. In the crystallizer (a) of the first stage crystallization unit, the acrylic acid solution was crystallized to obtain a suspension containing acrylic acid crystals. At this time, the crystallization temperature in the crystallizer (a) of the first stage crystallization unit was -3.0°C. The ratio of the mass of the acrylic acid crystals contained in the suspension to the mass of the suspension discharged from the crystallizer (a) of the first stage crystallization unit was 30 wt%.

The obtained suspension was supplied from the crystallizer (a) of the first stage crystallization unit to a solid-liquid separator (b) of the first stage crystallization unit. In the solid-liquid separator (b) of the first stage crystallization unit, the suspension obtained in the crystallizer (a) of the first stage crystallization unit was separated into acrylic acid crystals and a mother liquor. At this time, in the solid-liquid separator (b) of the first stage crystallization unit, solid-liquid separation of the suspension was performed through filtration. Meanwhile, the acrylic acid crystals separated in the solid-liquid separator (b) of the first stage crystallization unit were melted to obtain a concentrated acrylic acid solution.

The mother liquor separated in the solid-liquid separator (b) of the first stage crystallization unit was divided into a first mother liquor stream and a second mother liquor stream, and the first mother liquor stream and the second mother liquor stream were circulated to the crystallizer (a) of the first stage crystallization unit and a crystallizer (a) of a second stage crystallization unit, respectively. At this time, the mass flow rate ratio of the first mother liquor stream and the second mother liquor stream branched from the solid-liquid separator (b) of the first stage crystallization unit was 0.9:9.1.

Through the crystallization process performed in the crystallizer (a) of the second stage crystallization unit, a suspension containing acrylic acid crystals was obtained. At this time, the crystallization temperature in the crystallizer (a) of the second stage crystallization unit was -13°C. The ratio of the mass of the acrylic acid crystals contained in the suspension to the mass of the suspension discharged from the crystallizer (a) of the second stage crystallization unit was 30 wt%.

Thereafter, the obtained suspension was supplied from the crystallizer (a) of the second stage crystallization unit to a solid-liquid separator (b) of the second stage crystallization unit. In the solid-liquid separator (b) of the second stage crystallization unit, the suspension obtained in the crystallizer (a) of the second stage crystallization unit was separated into acrylic acid crystals and a mother liquor. At this time, in the solid-liquid separator (b) of the second stage crystallization unit, solid-liquid separation of the suspension was performed through filtration. Meanwhile, the acrylic acid crystals separated in the solid-liquid separator (b) of the second stage crystallization unit were melted to obtain a concentrated acrylic acid solution.

The mother liquor separated in the solid-liquid separator (b) of the second stage crystallization unit was divided into a first mother liquor stream and a second mother liquor stream, and the first mother liquor stream and the second mother liquor stream were supplied to the crystallizer (a) of the second stage crystallization unit and the outside of the system, respectively. At this time, the mass flow rate ratio of the first mother liquor stream and the second mother liquor stream branched from the solid-liquid separator (b) of the second stage crystallization unit was 1:1.

Meanwhile, a mixed stream was formed by the concentrated acrylic acid solution separated in the solid-liquid separator (b) of the second stage crystallization unit and the concentrated acrylic acid solution separated in the solid-liquid separator (b) of the first stage crystallization unit, and the mixed stream is obtained as purified acrylic acid.

Acrylic acid (96.6 wt%), acetic acid (1.3 wt%), and water (2.1 wt%) were contained in the purified acrylic acid, and the final yield of acrylic acid was 73.6%.

As a result, in Comparative Example 4, it was confirmed that the purity of the purified acrylic acid, that is, the content of acrylic acid contained in the purified acrylic acid, was the lowest.

### Comparative Example 5

Compared to Example 1, in Comparative Example 5, the crystallization temperature in the crystallizer of the first stage crystallization unit was -5°C, the mass flow rate ratio of the first mother liquor stream and the second mother liquor stream divided from the solid-liquid separator of the first stage crystallization unit was 4.4:5.6, and the mass flow rate ratio of the first mother liquor stream and the second mother liquor stream branched from the solid-liquid separator of the second stage crystallization unit was 2.5:7.5.

As a result, acrylic acid (99.0 wt%), acetic acid (0.57 wt%), and water (0.38 wt%) were contained in the purified acrylic acid, and the final yield of acrylic acid was 45.8%.

Compared to Example 1, Comparative Example 5 was a case in which the crystallization temperature in the crystallizer of the first stage crystallization unit was high, and it was confirmed that the purity of acrylic acid in the purified acrylic acid was the same, but the final yield of acrylic acid was significantly reduced, compared to Example 1.

### [Detailed Description of Main Elements]

(a): Crystallizer
(b): Solid-liquid separator

## Claims

1. A method for preparing acrylic acid, the method comprising a multi-stage crystallization process performed by two or more crystallization units that are connected in series and each includes a crystallizer and a solid-liquid separator, the method comprising:
in the crystallizer of each stage, crystallizing acrylic acid contained in an acrylic acid solution introduced into the crystallizer of each stage to obtain a suspension containing acrylic acid crystals, and supplying the suspension to the solid-liquid separator of each stage,
in the solid-liquid separator of each stage, separating the suspension into a solid and a liquid to obtain a concentrated acrylic acid solution and a mother liquor,
dividing the mother liquor divided into a first mother liquor stream and a second mother liquor stream, circulating the first mother liquor stream to a crystallizer at a front stage of a stage including the solid-liquid separator in which the mother liquor is separated or to a mother liquor treatment process, and circulating the second mother liquor stream to a crystallizer at the stage including the solid-liquid separator in which the mother liquor is separated,
introducing the concentrated acrylic acid solution into a crystallizer at a rear stage of the stage including the solid-liquid separator in which the concentrated acrylic acid solution is separated, and
obtaining purified acrylic acid from the solid-liquid separator of the rearmost stage crystallization unit among the two or more crystallization units,
wherein a first stage crystallization unit among the two or more crystallization units includes a crystallizer having a crystallization temperature of -20 to -10°C.

2. The method of claim 1, wherein a content of the acrylic acid contained in the acrylic acid solution introduced into the crystallizer of the first stage crystallization unit is 80 to 95 wt%.

3. The method of claim 1, wherein a ratio of the mass of the acrylic acid crystals contained in the suspension to the mass of the suspension discharged from the crystallizer of each stage is 10 to 40 wt%.

4. The method of claim 1, wherein a mass flow rate ratio of the first mother liquor stream and the second mother liquor stream is 0.5:9.5 to 9.5:0.5.

5. The method of claim 1, wherein the two or more crystallization units are including the first stage crystallization unit and a second stage crystallization unit,
a crystallization temperature in a crystallizer of the first stage crystallization unit is -20 to -10°C, and
a crystallization temperature in a crystallizer of the second stage crystallization unit is 0 to 12°C.

6. The method of claim 5, wherein a mass flow rate ratio of a first mother liquor stream and a second mother liquor stream branched from a solid-liquid separator of the first stage crystallization unit is 0.5:9.5 to 1:1, and
a mass flow rate ratio of a first mother liquor stream and a second mother liquor stream branched from a solid-liquid separator of the second stage crystallization unit is 1:1 to 9:1.

7. The method of claim 1, wherein the first stage crystallization unit includes an auxiliary crystallizer disposed at a front stage of the crystallizer.

8. The method of claim 7, wherein a crystallization temperature in the auxiliary crystallizer is -5 to 5°C.

9. The method of claim 1, wherein the acrylic acid solution introduced into the crystallizer of the first stage crystallization unit is obtained by bringing a mixed gas containing acrylic acid into contact with an absorption solvent.

10. The method of claim 9, wherein the mixed gas containing acrylic acid is a reaction product obtained by subjecting a reactant containing gas containing oxygen and a raw material compound to a gas phase oxidation reaction.

11. The method of claim 10, wherein the raw material compound is one or more compounds selected from the group consisting of propane, propylene, butane, i-butylene, t-butylene, and (meth)acrolein.

12. The method of claim 1, wherein the obtaining of the concentrated acrylic acid solution in the solid-liquid separator of each stage includes:
obtaining acrylic acid crystals by separating the suspension into a solid and a liquid in the solid-liquid separator of each stage; and
obtaining the concentrated acrylic acid solution by melting the acrylic acid crystals.

13. The method of claim 1, wherein the mother liquor treatment process includes one or more of a purge and an acrylic acid purification process.
